# EUROPEAN PATENT APPLICATION

(11) **EP 1 813 247 A1**
(43) Date of publication of application: **01.08.2007**
(21) Application number: 05813430.5
(22) Date of filing: 25.10.2005
(51) Int. Cl.: A61J 1/14

(54) **PROTECTIVE DEVICE FOR TRANSPORTING CLINICAL SAMPLES AND SIMILAR**

(30) Priority: 26.10.2004 ES 200402563
(71) Applicant: Sanchez Taboas, Jorge Horacio, E-36203 Vigo (ES); Sanchez Taboas, Ignacio Javier, E-36203 Vigo (ES); Sanchez Taboas, Marcos José, E-36203 Vigo (ES)
(72) Inventor: Sanchez Taboas, Jorge Horacio, E-36203 Vigo (ES); Sanchez Taboas, Ignacio Javier, E-36203 Vigo (ES); Sanchez Taboas, Marcos José, E-36203 Vigo (ES)
(74) Representative: Ungria Lopez, Javier
(86) International application number: PCT/ES2005/000572
(87) International publication number: WO 2006/048482

(57) **Abstract**

The invention relates to a protective device for transporting clinical samples and similar. The inventive device comprises a primary sample-carrying container, a secondary container in which the primary container is housed and a tertiary or outer container in which the secondary container is housed. According to the invention, the secondary container is equipped with a handle which is built into the same block or mould and the lid in order to prevent breakage and the build-up of dirt. The aforementioned lid comprises an integrated eutectic plate which has been specially designed for said device. The secondary container is produced using a triple wall production process, both by means of injection and rotation moulding, although there may be a single insulating wall external thereto.

## Description

### OBJECT OF THE INVENTION

As stated in the title of this descriptive specification, the present invention relates to a protective device for transporting clinical samples and similar, with the aim of ensuring the characteristics of the products found inside the device without them undergoing any unforeseen alterations.

The invention basically concerns certain improvements in the lids or devices corresponding to Utility Model Nos. U-200302641 and U-200401723, which are also owned by the same applicant as the present Patent Application which is now sought to register.

So, the device has been conceived in order to cover and protect the bodies of the primary containers, destined to facilitate the transport of organic samples, housed in an element capable of maintaining the sample-carrying elements in a defined and secure position, the invention being conceived as a protection element for a primary container of the sample itself, a secondary container, which retains the sample in a suitable and more secure position, on which is incorporated the tertiary container which will function as support for the primary and secondary containers, and also as an outer packaging for the unit.

The inventive device has application in the industry concerned with the manufacture of elements and accessories for the protection of containers, and especially for containers destined for the transporting of products that can be used as medical samples and similar.

### PRIOR ART OF THE INVENTION

There currently exist certain devices such as those corresponding to Utility Models Nos. U-200302641 and U-200401723, cited above.

The first of these basically comprises a rack supporting some tubes containing the product to transport, and at the same time the rack is housed in a rigid secondary container with a lid with a lock and a tertiary container with another locking lid.

The other later Utility Model No. U-200401723 consists of a device improved with respect to the first one, in which the secondary container is sealed, with the capacity to withstand internal pressure, and polygonal in cross-section.

In addition, it incorporates a tertiary or outer container of polygonal cross-section with a material interior for the cushioning of impacts for the perfect security of the secondary container, with a support for cooling devices and for documentation. Both are constructed in materials capable of withstanding autoclavage and/or chemical disinfection. Also, both containers will have their corresponding lid in a separate or folding piece.

Another characteristic relates to the fact that the tertiary container incorporates a safety valve for controlling pressure differences when air transport is used or transport with coolants of the dry ice type. It also incorporates supports for the reception of documentation and others, along with an absorbent material in the secondary recipient.

### DESCRIPTION OF THE INVENTION

The protective device for transporting clinical samples and similar constituting the object of the invention is defined on the basis of three bodies: a primary sample-carrying container, a secondary container and a tertiary or outer container.

The latter two are designed and manufactured especially in order to comply with the international legislation for the transport of clinical samples, whether or not infectious for human beings or for animals, culture samples for biological agents, whether or not pathogens, vaccines and organs for their transplant, study or research.

The invention is conceived as the manufacture of a set of secondary and tertiary containers, especially designed for meeting international legislation of the United Nations, the World Health Organisation (Global Health Security, Transport of Infectious Substances), legislation of the I.A.T.A. relating to the transport of dangerous substances (Dangerous Goods Regulations), the European Agreement Concerning the International Carriage of Dangerous Goods by Road (ADR); United States regulations published by the Department of Transportation (D.O.T. - Research and Special Programs Administration, 49 CFR), guides and recommendations published by Australia Post, the Multilateral M143 Agreement signed by Spain, and other reference standards and recommendations.

The invention is conceived as a tertiary container which acts as a perimetric belt for the secondary container, this tertiary container being sufficiently strong for resisting its handling under normal operations of the transport of samples between extraction centres, or filling of tubes and container of samples, and the analysis and/or research centres. The unit is conceived for resisting the conditions of pressure, temperature, vibrations and hygrometry to which it is subject during transport, whether this be by road, air, rail or sea.

The secondary container, unfailingly housed in the aforementioned tertiary or outer container, which will not be circular in cross-section, will have a polygonal cross-section of any number of sides with a minimum of three, having its pertinent base in the lower part and a lid in the upper part, which shall be able to be incorporated into the unit in a way that is folding or independent of the unit, which will permit the incorporation of the sample-carrying unit and/or racks, and which can be closed in order to protect the racks and the samples. This is especially conceived as the protector element of the actual sample on account of its special design and manufacture. It is conceived as a container capable of withstanding a differential internal pressure of 0.95 bar or greater, all this via the construction material and the manufacturing process, the latter being injection of plastic materials and moulding by rotation, though without being limited to this, and due also the use of specially designed seals and high-pressure locks.

This container has been manufactured in order to be capable of maintaining a controlled temperature inside, which can be positive or negative by means of cooling plates.

This set of primary container, with the sample, secondary and tertiary container has been designed and manufactured in order to pass every single one of the tests designed by existing legislation in relation to the transport of clinical samples, whether or not infectious for human beings or for animals, culture samples of biological agents, whether or not pathogens, vaccines and organs for their transplant, study or research. In particular, they are designed to pass drop tests, both from 1.2 metres and from 9 metres, puncture tests and other tests described in the legislation and recommendations at the international level. Included in this secondary container is sufficient absorbent material between the samples packed in the primary and in this secondary container, so that this material will absorb possible spillages in the event of impact or breakage thereof.

Below, in order to facilitate a better understanding of this specification and forming an integral part thereof, some figures are attached in which, on an illustrative rather than limiting basis, the most characteristic details of the invention have been represented.

### BRIEF DESCRIPTION OF THE DRAWINGS

**Figure 1.-** Shows a perspective view of the protective device for transporting clinical samples and similar, forming the inventive object. It basically shows a secondary container housed within another tertiary or outer container, with their respective lids in the open position.
**Figure 2.-** Shows a perspective view of the secondary container in the open position.

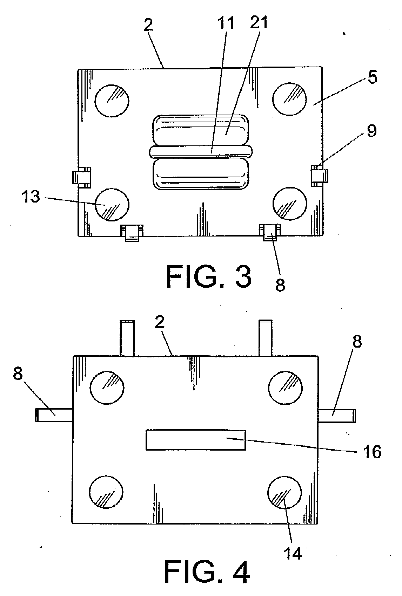

**Figure 3.-** Shows an upper plane view of the secondary container essentially showing the outer face of the lid.
**Figure 4.-** Shows a lower plane view of the container essentially showing the bottom on its outside part of the secondary container

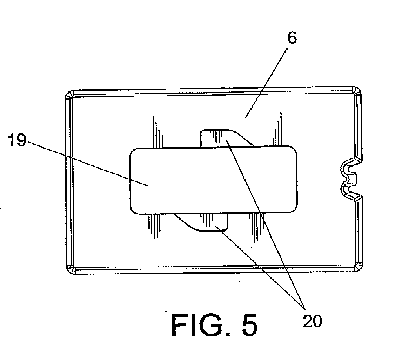

**Figure 5.-** Shows a view of an eutectic plate which is coupled to the lid of the secondary container.

### DESCRIPTION OF THE PREFERRED FORM OF EMBODIMENT

Considering the numbering adopted in the figures, the protective device for transporting clinical samples in principle comprises a primary container, not represented in the figures, which is the one that will carry the clinical samples, a secondary container 2 and a tertiary or outer container 1, the novelty being focused precisely on these latter two containers.

In a first embodiment, the secondary container 2 can be manufactured via a plastic injection process with a double wall incorporating a thermal insulation material or by means of a rotation moulding system.

The tertiary container 1, on the other hand, will be manufactured with materials, rigid or otherwise, performing the function of a protective cover with regard to outside handling during its transportation. And in the second version, the secondary container 2 being manufactured by injection of a single wall. Consequently, due to the secondary container 2 not having a double wall, the thermal control properties will be applied to the construction materials of the tertiary container 1.

The manufacturing materials can be diverse, and as such they may or may not be limited to products derived from petroleum, polyethylenes, polypropylenes, ABS, polycarbonate or other rigid metallic materials, such as aluminium for example, elements manufactured in non-rigid materials, such as polyethylenes, canvas or other non-rigid materials, these latter materials, canvas and non-rigid, having the main function of acting as outer packaging when so conceived.

All this shapes the perimetric belt of the body of the container for clinical samples whether or not infectious for human beings or for animals, culture samples of biological agents, whether or not pathogens, vaccines and organs for their transplant, study or research, which is introduced into the secondary container 2, in supports or racks, or directly into the sample-carrying container, and the latter is in turn introduced into the tertiary container 1. All with their corresponding lids in order to prevent the leakage of liquid or solid organic matter. The secondary container 2 incorporates a lid referenced with number 5, while the tertiary container incorporates a lid referenced with number 17.

So, the inventive device consists of certain improvements to the secondary and tertiary containers protected by previous Utility Models Nos. U-200302641 and U-200401723, both on the outer zone of the sides and on the upper and lower zone of a container for samples of biological products. Added as fundamental improvements are the incorporation into the unit of greater security and thermal control of temperatures, due to the introduction of double wall materials, combined with the protective cover for the tertiary container 1, or the simple wall with thermal control designed in the tertiary container, with eutectic plates 6 in the lid 5 for the distribution of cold from the upper part to the lower, especially designed for maintaining samples at a temperature below room temperature. The special high-pressure locks and the sealing O-rings 7 and/or seals with a saw-tooth cross-section have been specially designed for this secondary container 2 in order to thereby increase the tightening in pressure tests.

Indeed, a primary container holding the actual sample will be deposited in the improved secondary container 2 in racks or directly, in such a way that if the primary containers are fragile, being made of glass or other materials, there does not exist any contact at all between the different primary containers, thereby preventing them from being able to break or be pierced and their contents spilling onto the secondary container 2.

This secondary container 2 will be housed in the tertiary or outer container 1 in such a way that a filling material is interposed, this material being able to be incorporated independently or as a structural part of the tertiary container 1, in order to provide a cushion against possible impacts or blows.

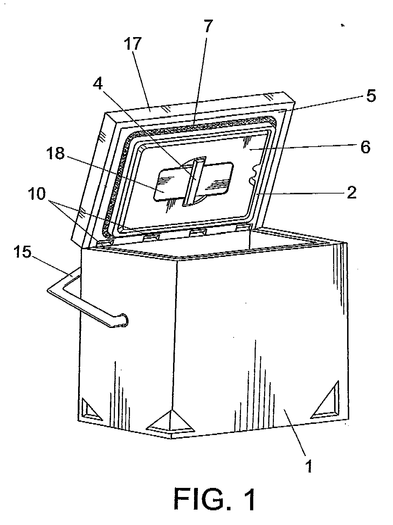

Figure 1 shows the unit of the secondary container 2 and the tertiary container 1 with their handles 15 and the eutectic plate 6, which is incorporated into the lid 5 of the secondary container 2.

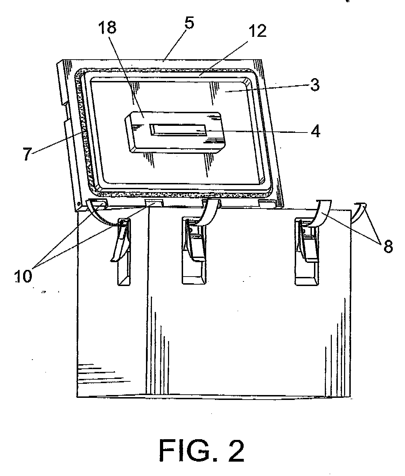

Represented in figure 2 is a secondary container 2 with its respective lid 5, the space 3 and the means of coupling 4 in order to secure the eutectic plate 6, the seal 7, and the locking elements 8 for the lid 5. It also shows a hinge 10 and a perimetric projection or fillet which demarcates a recess in the lid 5 in order to house the eutectic plate.

Moreover, the lid incorporates some clamps 9 for ensuring its airtight seal by means of the locking elements 8 which will be coupled precisely in those clamps 9, this lid 5 furthermore incorporating a central handle 11 integral with the actual lid along with some engaging projections 13. In correspondence with the handle 11 there exists a characteristic and wide central concavity 21 in the lid 5 of the secondary container 2.

In the bottom or base of the secondary container 2 on its outer face there can be seen some depressions 14 complementary with the projections 13 of the lid 5 and of the handle 11 in order to facilitate the correct stacking of several containers. It can also be seen that the bottom incorporates a central depression 16 for housing the handle 11, which projects slightly with respect to the outer face of the lid.

The eutectic plate basically comprises a flat body with a central window 19 complementary with a projecting portion 18 established on the inner face of the lid 5 of the secondary container 2. Moreover, this plate incorporates some areas in bas-relief 20 in order to facilitate the securing of said plate in the lid 5 of the secondary container 2 by means of the end sections of the coupling element 4.

The device as a whole is conceived as a container capable of maintaining the contents of the primary container perfectly refrigerated, said container not being represented in the figures as referred to already.

## Claims

1. **PROTECTIVE DEVICE FOR TRANSPORTING CLINICAL SAMPLES AND SIMILAR**, which comprises a primary sample-carrying container, a secondary container with a lid in which the primary container is housed and a tertiary or outer container with a lid in which the secondary container is housed; **characterised in that** the lid (5) of the secondary container (2) provides a sealed lock with the aid of an airtight seal (7), in such a way that when the lid (5) is closed against the opening of this secondary container (2) the airtight seal (7) is pressed by means of locking elements (8), these locking elements being provided in depressions of the side faces of the secondary container (2) in proximity to its opening; the secondary container (2) furthermore including a eutectic plate (6).

2. **PROTECTIVE DEVICE FOR TRANSPORTING CLINICAL SAMPLES AND SIMILAR,** according to claim 1, **characterised in that** the airtight seal (7) is arranged in a closed channel of the inner face of the lid (5) in proximity to its perimetric border.

3. **PROTECTIVE DEVICE FOR TRANSPORTING CLINICAL SAMPLES AND SIMILAR,** according to claim 1, **characterised in that** the airtight seal (7) is arranged in a closed channel provided in the opening of the secondary container (2).

4. **PROTECTIVE DEVICE FOR TRANSPORTING CLINICAL SAMPLES AND SIMILAR**, according to any of the above claims, **characterised in that** the eutectic plate (6) is located in a complementary space (3) demarcated by a perimetric projection (12) made in the inner face of the lid (5), including certain means of securing of said eutectic plate (6) to the lid (5).

5. **PROTECTIVE DEVICE FOR TRANSPORTING CLINICAL SAMPLES AND SIMILAR,** according to claim 4, **characterised in that** the eutectic plate (6) comprises a flat body with a central window (19) which is complemented with a projecting portion (18) of the inner face of the lid (5) which forms part of the secondary container (2).

6. **PROTECTIVE DEVICE FOR TRANSPORTING CLINICAL SAMPLES AND SIMILAR,** according to claims 4 and 5, **characterised in that** means of securing of the plate (6) to the lid (5) consist of a rotating arm (4) coupled in a central point in correspondence with the projecting portion (18), in such a way that in the attached position, the end sections of this arm (4) act as a stop against some small areas in bas-relief (20) of the eutectic plate (6).

7. **PROTECTIVE DEVICE FOR TRANSPORTING CLINICAL SAMPLES AND SIMILAR,** according to any of claims 1 to 3, **characterised in that** the airtight seal (7) is capable of supporting a differential internal pressure of at least 0.95 bar when the locking elements (8) are activated.

8. **PROTECTIVE DEVICE FOR TRANSPORTING CLINICAL SAMPLES AND SIMILAR,** according to claim 1, **characterised in that** the lid (5) of the secondary container (2) incorporates some small depressions (9) for clamping of the locking elements (8).

9. **PROTECTIVE DEVICE FOR TRANSPORTING CLINICAL SAMPLES AND SIMILAR,** according to claim 1, **characterised in that** the lid (5) of the secondary container (2) is coupled thereto by means of hinged elements (10).

10. **PROTECTIVE DEVICE FOR TRANSPORTING CLINICAL SAMPLES AND SIMILAR,** according to claim 1, **characterised in that** the secondary container (2) incorporates a central handle (11) which is integral with the folding lid (5), and at the same time it comprises an arm located above a wide concavity (21) made in the outer face of said lid (5).

11. **PROTECTIVE DEVICE FOR TRANSPORTING CLINICAL SAMPLES AND SIMILAR,** according to claim 1, **characterised in that** the outer face of the lid (5) of the secondary container (2) comprises some projections (13) which are complemented with some depressions (14) made in the bottom of said container (2) for its correct stacking, this bottom furthermore including another elongated depression (16) which is complemented with the handle (11) which forms an integral part of the lid (5) of the secondary container (2).

12. **PROTECTIVE DEVICE FOR TRANSPORTING CLINICAL SAMPLES AND SIMILAR,** according to claim 1, **characterised in that** the tertiary container includes a cushioned inner part made of an impact-proof material, such as polystyrene foam or polyethylene for example, and an outer material that is rigid or otherwise; all this in order to protect the device against possible impacts.

13. **PROTECTIVE DEVICE FOR TRANSPORTING CLINICAL SAMPLES AND SIMILAR,** according to claim 1, **characterised in that** the tertiary container (1) incorporates handles (15) for transportation.

14. **PROTECTIVE DEVICE FOR TRANSPORTING CLINICAL SAMPLES AND SIMILAR,** according to claim 1, **characterised in that** it incorporates an autonomous refrigeration system for cooling and maintenance of the inside temperature.
